# EUROPÄISCHE PATENTSCHRIFT

(11) **EP 3 122 316 B1**
(45) Veröffentlichungstag und Bekanntmachung des Hinweises auf die Patenterteilung: **27.06.2018**
(21) Anmeldenummer: 15701354.1
(22) Anmeldetag: 27.01.2015
(51) Int. Cl.: A61K 8/35, A61K 8/36, A61K 8/44, A61K 8/55, A61Q 17/04, A61Q 19/00

(54) **ÖL IN WASSER-EMULSIONEN MIT EINEM GEHALT AN 4-HYDROXYACETOPHENON UND ANIONISCHEN EMULGATOREN**
OIL-IN-WATER EMULSIONS CONTAINING 4-HYDROXYACETOPHENONE AND ANIONIC EMULSIFIERS
ÉMULSIONS DE TYPE AQUEUX CONTENANT DU 4-HYDROXYACÉTOPHÉNONE ET DES ÉMULSIFIANTS ANIONIQUES

(30) Priorität: 26.03.2014 DE 102014104255
(43) Veröffentlichungstag der Anmeldung: 01.02.2017
(73) Patentinhaber: Symrise AG, 37603 Holzminden (DE)
(72) Erfinder: PRUNS, Julia, 20144 Hamburg (DE); RASCHKE, Thomas, 25421 Pinneberg (DE); NISSEN, Bente, 20144 Hamburg (DE)
(74) Vertreter: Fabry, Bernd
(86) Internationale Anmeldenummer: PCT/EP2015/051554
(87) Internationale Veröffentlichungsnummer: WO 2015/144333

(56) Entgegenhaltungen:
- EP-A1- 2 774 481
- EP-A1- 2 774 604
- DE-A1-102010 063 888
- JP-A- H07 206 645
- KR-A- 20130 134 976
- US-A1- 2007 269 390
- SYMRISE: "Multiple Benefits for Cosmetics with SymSave H", INTERNET CITATION, 26. Juli 2013 (2013-07-26), XP007923019, Gefunden im Internet: URL:http://www.symrise.com/newsroom/articl e/multiple-benefits-for-cosmetics-with-sym saveR-h/ [gefunden am 2015-02-25]
- RAJABI L ET AL: "Acetophenones with selective antimycobacterial activity", LETTERS IN APPLIED MICROBIOLOGY, OXFORD, GB , Bd. 40, Nr. 3 1. März 2005 (2005-03-01), Seiten 212-217, XP002693043, ISSN: 1472-765X, DOI: 10.1111/J.1472-765X.2005.01657.X Gefunden im Internet: URL:http://onlinelibrary.wiley.com/doi/10. 1111/j.1472-765X.2005.01657.x/full [gefunden am 2013-02-18]

## Beschreibung

Die vorliegende Erfindung betrifft kosmetische bzw. dermatologische Zubereitungen, enthaltend Wirkstoffe zum Schutze der empfindlichen Haut vor Irritationen sowie die Verwendung solcher Wirkstoffe und Kombinationen solcher Wirkstoffe auf dem Gebiete der kosmetischen und dermatologischen Hautpflege. In einer vorteilhaften Ausführungsform betrifft die vorliegende Erfindung eine Anwendung, welche es erlaubt, die Stabilität von Emulsionen, Gelen, wässrigen Zubereitungen oder Fett-Formulierungen zu steigern.

Die äußerste Schicht der Epidermis, das Stratum corneum (Hornschicht), ist als wichtige Barriereschicht von besonderer Bedeutung u.a. für den Schutz vor Umwelteinflüssen und Austrocknung. Die Hornschicht wird im Kontakt mit der Umwelt ständig abgenutzt und muss deshalb ununterbrochen erneuert werden.

Ein heute in der Fachwelt weitverbreitetes Hautmodell fasst das Stratum corneum als ZweiKomponenten-System, ähnlich einer Ziegelsteinmauer (Ziegelstein-Mörtel-Modell), auf. In diesem Modell entsprechen die Korneozyten (Hornzellen) den Ziegelsteinen, die kompliziert zusammengesetzte Lipidmembran in den Interzellularräumen entspricht dem Mörtel.

Außer ihrer Barrierewirkung gegen externe chemische und physikalische Einflüsse tragen die epidermalen Lipide auch zum Zusammenhalt der Hornschicht bei und haben Einfluss auf die Hautglätte. Im Gegensatz zu den Talgdrüsenlipiden, die keinen geschlossenen Film auf der Haut ausbilden, sind die epidermalen Lipide über die gesamte Hornschicht verteilt.

Das äußerst komplexe Zusammenwirken der feuchtigkeitsbindenden Substanzen und der Lipide der oberen Hautschichten ist für die Regulation der Hautfeuchte sehr wichtig. Daher enthalten Kosmetika in der Regel, neben ausgewogenen Lipidabmischungen und Wasser, wasserbindende Substanzen.

Neben der chemischen Zusammensetzung ist jedoch auch das physikalische Verhalten dieser Substanzen von Bedeutung. Daher ist die Entwicklung von sehr gut bioverträglichen Emulgatoren bzw. Tensiden mit flüssigkristallinen Eigenschaften wünschenswert. Damit formulierte Produkte unterstützen die flüssigkristalline Organisation der Interzellularlipide des Stratum Corneums und verbessern so die Barriereeigenschaften der Hornschicht. Besonders vorteilhaft ist es, wenn deren Molekülbestandteile aus natürlicherweise in der Epidermis vorkommenden Substanzen bestehen.

Unter kosmetischer Hautpflege ist in erster Linie zu verstehen, dass die natürliche Funktion der Haut als Barriere gegen Umwelteinflüsse (z.B. Schmutz, Chemikalien, Mikroorganismen) und gegen den Verlust von körpereigenen Stoffen (z.B. Wasser, natürliche Fette, Elektrolyte) gestärkt oder wiederhergestellt wird.

Wird diese Funktion gestört, kann es zu verstärkter Resorption toxischer oder allergener Stoffe oder zum Befall von Mikroorganismen und als Folge zu toxischen oder allergischen Hautreaktionen kommen.

Ziel der Hautpflege ist es ferner, den durch tägliches Waschen verursachten Fett- und Wasserverlust der Haut auszugleichen. Dies ist gerade dann wichtig, wenn das natürliche Regenerationsvermögen nicht ausreicht. Außerdem sollen Hautpflegeprodukte vor Umwelteinflüssen, insbesondere vor Sonne und Wind, schützen und die Hautalterung verzögern.

Medizinische topische Zusammensetzungen enthalten in der Regel ein oder mehrere Medikamente in wirksamer Konzentration. Der Einfachheit halber wird zur sauberen Unterscheidung zwischen kosmetischer und medizinischer Anwendung und entsprechenden Produkten auf die gesetzlichen Bestimmungen der Bundesrepublik Deutschland verwiesen (z.B. Kosmetikverordnung, Lebensmittel- und Arzneimittelgesetz).

Übliche kosmetische Darreichungsformen sind Emulsionen, also metastabile Zwei- oder Mehrphasensysteme bei welchen die einzelnen Phasen im flüssigen Zustande vorliegen. Die gängigsten Emulsionen sind O/W- und W/O-Emulsionen. Seltenere Darreichungsformen sind multiple Emulsionen, also solche, welche in den Tröpfchen der dispergierten (oder diskontinuierlichen) Phase ihrerseits Tröpfchen einer weiteren dispergierten Phase enthalten, z.B. W/O/W-Emulsionen und O/W/O-Emulsionen.

Um die Metastabilität von Emulsionen gewährleisten zu können, sind in der Regel grenzflächenaktive Substanzen, also Emulgatoren, nötig.

An sich ist die Verwendung der üblichen kosmetischen Emulgatoren völlig unbedenklich. Dennoch können Emulgatoren, wie letztlich jede chemische Substanz, im Einzelfalle allergische oder auf Überempfindlichkeit des Anwenders beruhende Reaktionen hervorrufen.

So ist bekannt, dass bei manchen besonders empfindlichen Personen bestimmte Lichtdermatosen durch gewisse Emulgatoren und gleichzeitige Einwirkung von Sonnenlicht ausgelöst werden.

Es ist möglich, emulgatorfreie Zubereitungen herzustellen, welche beispielsweise in einer wässrigen Phase dispergierte Öltröpfchen, ähnlich einer O/W-Emulsion, aufweisen. Voraussetzung dafür kann sein, dass die kontinuierliche wässrige Phase ein die dispergierte Phase stabilisierendes Gelgerüst aufweist und andere Umstände mehr. Solche Systeme werden gelegentlich Hydrodispersionen oder Oleodispersionen genannt, je nachdem, welches die disperse und welches die kontinuierliche Phase darstellt.

Es ist für die kosmetische Galenik aber weder nötig noch möglich, auf Emulgatoren ganz zu verzichten, zumal eine gewisse Auswahl an besonders milden Emulgatoren existiert. Allerdings besteht ein Mangel des Standes der Technik an einer befriedigend großen Vielfalt solcher Emulgatoren, welche dann auch das Anwendungsspektrum entsprechend milder und hautverträglicher kosmetischer Zubereitungen deutlich verbreitern würde.

So war eine Aufgabe der vorliegenden Erfindung, kosmetische bzw. dermatologische Zubereitungen mit hervorragenden hautpflegenden Eigenschaften zur Verfügung zu stellen.

Ein Nachteil insbesondere von O/W-Emulsionen ist oft deren mangelnde Stabilität gegenüber höheren Elektrolytkonzentrationen, was sich in Phasentrennung äußert. Dies kann zwar auch bei W/O-Emulsionen gelegentlich zu Problemen führen, tritt dort aber bei weitem nicht so in den Vordergrund wie bei O/W-Systemen. Zwar lässt sich diesen oft durch geeignete Wahl des Emulgatorsystems in gewissem Maße Abhilfe schaffen, es treten dann aber ebenso oft andere Nachteile auf.

Es ist andererseits oft wünschenswert, bestimmte Elektrolyte einzusetzen, um deren sonstige physikalische, chemische bzw. physiologische Eigenschaften nutzen zu können.

Ferner setzen erhöhte Schwermetallkonzentrationen - herstellungsbedingt durch Fertigung in stählernen Behältern, Rührwerken, Leitungen usw. - die Stabilität kosmetischer Zubereitungen erheblich herab. Durch die Komplexierung von störenden Metallen wie Mn, Fe, Cu und anderer können beispielsweise unerwünschte chemische Reaktionen in kosmetischen oder dermatologischen Zubereitungen verhindert werden.

Komplexbildner, insbesondere Chelatoren, bilden mit Metallatomen Komplexe, welche bei Vorliegen eines oder mehrerer mehrbasiger Komplexbildner, also Chelatoren, Metallacyclen darstellen. Chelate stellen Verbindungen dar, in denen ein einzelner Ligand mehr als eine Koordinationsstelle an einem Zentralatom besetzt. In diesem Falle werden also normalerweise gestreckte Verbindungen durch Komplexbildung über ein Metall-Atom od. -Ion zu Ringen geschlossen. Die Zahl der gebundenen Liganden hängt von der Koordinationszahl des zentralen Metalls ab. Voraussetzung für die Chelatbildung ist, dass die mit dem Metall reagierende Verbindung zwei oder mehr Atomgruppierungen enthält, die als Elektronendonatoren wirken.

Nachteilig an vielen Chelatoren ist, dass sie biologisch nur schwer abbaubar sind, nahezu zwangsläufig in den ökologischen Wasserkreislauf eingetragen werden. Durch Solubilisierung ausgefällter bzw. im Sediment gebundener - und dadurch an sich relativ unschädlicher - Schwermetallionen können sie im schlimmsten Falle zu deren Reaktivierung beitragen.

Eine weitere Aufgabe der vorliegenden Erfindung war es also, Lösungswege zu kosmetischen oder dermatologischen Emulsionen, insbesondere O/W-Emulsionen, aufzudecken, welche gegenüber erhöhten Elektrolytkonzentrationen bzw. erhöhten lonenstärken bzw. Schwermetallionen stabil sind.

Übliche, und sich gerade in neuerer Zeit immer weiter verbreitende kosmetische und dermatologische Zubereitungsformen sind Gele.

Im technischen Sinne werden unter Gelen verstanden: Relativ formbeständige, leicht verformbare disperse Systeme aus zumindest zwei Komponenten, welche in der Regel aus einem - meist festen - kolloid zerteilten Stoff aus langkettigen Molekülgruppierungen (z.B. Gelatine, Kieselsäure, Polysaccharide) als Gerüstbildner und einem flüssigen Dispersionsmittel (z.B. Wasser) bestehen. Der kolloidal zerteilte Stoff wird oft als Verdickungs- oder Geliermittel bezeichnet. Er bildet ein räumliches Netzwerk im Dispersionsmittel, wobei einzelne kolloidal vorliegende Partikel über elektrostatische Wechselwirkung miteinander mehr oder weniger fest verknüpft sein können. Das Dispersionsmittel, welches das Netzwerk umgibt, zeichnet sich durch elektrostatische Affinität zum Geliermittel aus, d.h., ein vorwiegend polares (insbesondere: hydrophiles) Geliermittel geliert vorzugsweise ein polares Dispersionsmittel (insbesondere: Wasser), wohingegen ein vorwiegend unpolares Geliermittel vorzugsweise unpolare Dispersionsmittel geliert.

Starke elektrostatische Wechselwirkungen, welche beispielsweise in Wasserstoffbrückenbindungen zwischen Geliermittel und Dispersionsmittel, aber auch zwischen Dispersionsmittelmolekülen untereinander verwirklicht sind, können zu starker Vernetzung auch des Dispersionsmittels führen. Hydrogele können zu fast 100 % aus Wasser bestehen (neben beispielsweise ca. 0,2 - 1,0 % eines Geliermittels) und dabei durchaus feste Konsistenz besitzen. Der Wasseranteil liegt dabei in eisähnlichen Strukturelementen vor, so dass Gele daher ihrer Namensherkunft [aus lat. "gelatum" = "Gefrorenes" über den alchimistischen Ausdruck "gelatina" (16. Jhdt.) für nhdt. "Gelatine"] durchaus gerecht werden.

In der kosmetischen und pharmazeutischen Galenik sind ferner auch Lipogele und Oleogele (aus Wachsen, Fetten und fetten Ölen) sowie Carbogele (aus Paraffin oder Petrolatum) geläufig. In der Praxis unterscheidet man Oleogele, welche praktisch wasserfrei vorliegen, Hydrogele, welche praktisch fettfrei sind. Meistens sind Gele durchsichtig. In der kosmetischen bzw. pharmazeutischen Galenik zeichnen sich Gele in aller Regel durch halbfeste, oft fließfähige Konsistenz aus.

Ferner sind sogenannte Tensidgele gebräuchliche Zubereitungen des Standes der Technik. Darunter versteht man Systeme, die neben Wasser eine hohe Konzentration an Emulgatoren aufweisen, typischerweise mehr als ca. 25 Gew.-%, bezogen auf die Gesamtzusammensetzung. Solubilisiert man in diese Tensidgele, fachsprachlich auch "surfactant gels" genannt, Ölkomponenten, werden Mikroemulsionsgele erhalten, welche auch als "ringing gels" bezeichnet werden. Durch Zusatz von nichtionischen Emulgatoren, beispielsweise Alkylpolyglycosiden, lassen sich kosmetisch elegantere Mikroemulsionsgele erhalten.

Zahlreiche kosmetische und dermatologische Wirkstoffe sind bekannt, darunter wasserlösliche - die sich demzufolge vorrangig in der Wasserphase einer Emulsion aufhalten - und öllösliche - die sich demzufolge vorrangig in der Ölphase einer Emulsion aufhalten.

Der log P Wert gibt den logarithmischen Koeffizienten des Verteilungskoeffizienten Octanol/Wasser K_{OW} an und ist ein Maß dafür, wie das Verhältnis zwischen Lipophilie (Fettlöslichkeit) und Hydrophilie (Wasserlöslichkeit) einer Substanz ist. Er dient als Modellabschätzung, in welcher Phase (Öl oder Wasser) sich die Substanz bevorzugt löst bzw. anreichert.

Entsprechend ist der log P positiv für lipophile Substanzen und negativ für hydrophile Substanzen.

So bedeutet ein log P-Wert von drei, dass sich der Wirkstoff in einer Octanol/Wasser Mischung im Verhältnis 1000:1 verteilen würde. Also um den Faktor 1000 höher angereichert in der Ölphase. Gleiches gilt für Substanzen, die nur eine sehr geringe Wasserlöslichkeit besitzen. Auch diese Substanzen sind - wenn sie kristallfrei formuliert werden - zum überwiegenden Teil in der Lipidphase zu finden.

Als Wirkstoffe, die sich zum überwiegenden Teil in der Ölphase aufhalten, werden im Rahmen dieser Anmeldung Substanzen verstanden, die
a) eine log P-Wert ≥ 2 haben oder
b) eine Wasserlöslichkeit ≤ 2.5g/L besitzen.

Bei Pflanzenextrakten bedeutet dass, dass 0,25%ige Lösungen nicht klar gelöst werden können.

Kosmetische und pharmazeutische Wirkstoffe sind oft, aber beileibe nicht immer, gegen Umwelteinflüsse stabil. So sind zahlreiche Instabilitäten gegenüber Sauerstoff - bzw. ganz allgemein Redoxprozesse - und UV-Licht, aber auch gegen Wärme und Anderes bekannt.

Zu den kosmetischen Inhaltstoffen, die bekanntermaßen empfindlich gegenüber derlei Prozessen sind, zählen natürliche Pflanzenöle mit einem Anteil an ungesättigten Fettsäuren (z.B. Sonnenblumenöl und Nachtkerzensamenöl), Vitamin A und seine Derivate, Vitamin C und seine Derivate, Pflanzenextrakte mit einem Anteil an Polyphenolen wie zum Beispiel Grüner Tee und Süßholz, organische Farbstoffe sowie synthetische Polymere aus der Gruppe der Polyacrylate.

Ein bekanntes und hochwirksames Antioxidans ist das 4-Hydroxyacetophenon, welches unter anderem von der Gesellschaft Symrise unter der Handelsbezeichnung "SymSave® H" verkauft wird. Es hat die CAS-Nr. 99-93-4 und zeichnet sich durch folgende chemische Struktur aus:

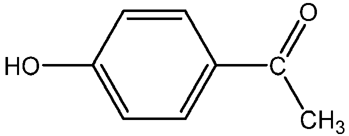

Es hat sich überraschend gezeigt, und darin liegt die Lösung dieser Aufgaben, dass die Verwendung von 4-Hydroxyacetophenon zum Erzielen oder Steigern der Stabilität kosmetischer Zubereitungen welche in Form von O/W-Emulsionen vorliegen, den Nachteilen des Standes der Technik abhelfen.

Der Begriff "Erzielen oder Steigern der Stabilität" bedeutet, dass eine entsprechende Rezeptur, die sich durch einen Gehalt an 4-Hydroxyacetophenon auszeichnet, eine höhere Stabilität an den Tag legt, als eine entsprechende Rezeptur ohne 4-Hydroxyacetophenon.

Dabei wird verglichen eine Rezeptur, die sich durch einen Gehalt an 4-Hydroxyacetophenon aufweist, mit einer solchen, bei der der Gewichtsanteil an 4-Hydroxyacetophenon durch einen entsprechenden Gewichtsanteil an Wasser oder der Hauptölkomponente der Zubereitung ersetzt worden ist.

Der Begriff "Stabilität" soll in diesem Zusammenhange bedeuten, dass das Produkt mit 4-Hydroxyacetophenon eine längere Zeit und/oder bei höheren Temperaturen gegen Phasentrennung geschützt ist als ein Produkt ohne 4-Hydroxyacetophenon.

Der Begriff "Stabilität" kann ebenfalls bedeuten, dass das Produkt mit 4-Hydroxyacetophenon, wenn es einen Farbstoff enthält, dieser Farbstoff eine längere Zeit und/oder bei höheren Temperaturen und/oder bei einer höhere Lichtdosis gegen chemische Zersetzung geschützt ist als ein Produkt ohne 4-Hydroxyacetophenon.

Der Begriff "Stabilität" kann ebenfalls bedeuten, dass das Produkt mit 4-Hydroxyacetophenon, wenn es einen Parfumbestandteil bzw. eine kosmetische Duftkomponente enthält, dieser Wirkstoff eine längere Zeit und/oder bei höheren Temperaturen und/oder bei einer höhere Lichtdosis gegen chemische Zersetzung geschützt ist als ein Produkt ohne 4-Hydroxyacetophenon.

Der Begriff "Stabilität" kann ebenfalls bedeuten, dass das Produkt mit 4-Hydroxyacetophenon, wenn es einen kosmetischen oder dermatologischen Wirkstoff enthält, dieser Wirkstoff eine längere Zeit und/oder bei höheren Temperaturen und/oder bei einer höhere Lichtdosis gegen chemische Zersetzung geschützt ist als ein Produkt ohne 4-Hydroxyacetophenon.

Es war für den Fachmann ferner nicht vorauszusehen gewesen, dass die erfindungsgemäßen Zubereitungen höhere Stabilität aufweisen und sich durch bessere Bioverträglichkeit auszeichnen würden als die Zubereitungen des Standes der Technik.

Zubereitungen gemäß der Erfindung sind vorteilhaft durch einen Gehalt von 0,01 bis 10 Gew.-%, insbesondere 0,05 bis 3,0 Gew.-%, an 4-Hydroxyacetophenon, insbesondere die 4-Hydroxyacetophenon, gekennzeichnet, jeweils bezogen auf das Gesamtgewicht der Zusammensetzung.

Es ist besonders vorteilhaft im Sinne der vorliegenden Erfindung, den Gehalt an üblichen Komplexbildnern, etwa solchen gewählt aus der Gruppe Ethylendiamintetraessigsäure (EDTA) und deren Anionen, Nitrilotriessigsäure (NTA) und deren Anionen, Hydroxyethylendiaminotriessigsäure (HOEDTA) und deren Anionen, Diethylenaminopentaessigsäure (DPTA) und deren Anionen, trans-1,2-Diaminocyclohexantetraessigsäure (CDTA) und deren Anionen gering zu halten bzw. auf diese ganz zu verzichten. Jedenfalls sollte ein Gehalt von ca. 0,5 Gew.-%, bezogen auf das Gesamtgewicht der kosmetischen Zubereitungen, an solchen Komplexbildnern bevorzugt nicht überschritten werden.

Die Herstellung von Zubereitungen gemäß der Erfindung geschieht nach den üblichen, dem Fachmanne geläufigen Regeln.

Es ist möglich und vorteilhaft, 4-Hydroxyacetophenon zu Beginn des Herstellprozesses der Wasserphase oder der Ölphase zuzuführen. Allerdings ist die Löslichkeit von 4-Hydroxyacetophenon in der Wasserphase und insbesondere in der Ölphase limitiert.

Die kosmetischen Zubereitungen gemäß der Erfindung können kosmetische Hilfsstoffe enthalten, wie sie üblicherweise in solchen Zubereitungen verwendet werden, z.B. Konservierungsmittel, Bakterizide, desodorierend wirkende Substanzen, Antitranspirantien, Insektenrepellentien, Vitamine, Mittel zum Verhindern des Schäumens, Farbstoffe, Pigmente mit färbender Wirkung, Verdickungsmittel, weichmachende Substanzen, anfeuchtende und/oder feucht haltende Substanzen, Fette, Öle, Wachse oder andere übliche Bestandteile einer kosmetischen Formulierung wie Alkohole, Polyole, Polymere, Schaumstabilisatoren, Elektrolyte, organische Lösungsmittel oder Silikonderivate.

Vorteilhaft können Zubereitungen gemäß der Erfindung außerdem Substanzen enthalten, die UV-Strahlung im UVB-Bereich absorbieren, wobei die Gesamtmenge der Filtersubstanzen z.B. 0,1 Gew.-% bis 30 Gew.-%, vorzugsweise 0,5 bis 10 Gew.-%, insbesondere 1,0 bis 6,0 Gew.-% beträgt, bezogen auf das Gesamtgewicht der Zubereitungen, um kosmetische Zubereitungen zur Verfügung zu stellen, die das Haar bzw. die Haut vor dem gesamten Bereich der ultravioletten Strahlung schützen. Sie können auch als Sonnenschutzmittel fürs Haar dienen.

Enthalten die Zubereitungen gemäß der Erfindung UVB-Filtersubstanzen, können diese öllöslich oder wasserlöslich sein. Erfindungsgemäß vorteilhafte öllösliche UVB-Filter sind z.B.:
- 3-Benzylidencampher-Derivate, vorzugsweise 3-(4-Methylbenzyliden)campher, 3-Benzylidencampher;
- 4-Aminobenzoesäure-Derivate, vorzugsweise 4-(Dimethylamino)-benzoesäure(2-ethylhexyl)ester, 4-(Dimethylamino)benzoesäureamylester;
- Ester der Zimtsäure, vorzugsweise 4-Methoxyzimtsäure(2-ethylhexyl)ester, 4-Methoxyzimtsäureisopentylester;
- Ester der Salicylsäure, vorzugsweise Salicylsäure(2-ethylhexyl)ester, Salicylsäure(4-isopropylbenzyl)ester, Salicylsäurehomomenthylester,
- Derivate des Benzophenons, vorzugsweise 2-Hydroxy-4-methoxybenzophenon, 2-Hydroxy-4-methoxy-4'-methylbenzophenon, 2,2'-Dihydroxy-4-methoxybenzophenon;
- Ester der Benzylmalonsäure, vorzugsweise 4-Methoxybenzylmalonsäuredi(2-ethylhexyl)ester,
- 2,4,6-Trianilino-(p-carbo-2'-ethyl-1'-hexyloxy)-1,3,5-triazin.

Vorteilhafte wasserlösliche UVB-Filter sind z.B.:
- Salze der 2-Phenylbenzimidazol-5-sulfonsäure wie ihr Natrium-, Kalium- oder ihr Triethanolammonium-Salz, sowie die Sulfonsäure selbst;
- Sulfonsäure-Derivate von Benzophenonen, vorzugsweise 2-Hydroxy-4-methoxybenzophenon-5-sulfonsäure und ihre Salze;
- Sulfonsäure-Derivate des 3-Benzylidencamphers, wie z.B. 4-(2-Oxo-3-bornylidenmethyl)benzolsulfonsäure, 2-Methyl-5-(2-oxo-3-bornylidenmethyl)sulfonsäure und ihre Salze sowie das 1,4-di(2-oxo-10-Sulfo-3-bornylidenmethyl)-Benzol und dessen Salze (die entsprechenden 10-Sulfato-verbindungen, beispielsweise das entsprechende Natrium-, Kalium- oder Triethanolammonium-Salz), auch als Benzol-1,4-di(2-oxo-3-bornylidenmethyl-10-Sulfonsäure bezeichnet.

Die Liste der genannten UVB-Filter, die gemäß der Erfindung verwendet werden können, soll selbstverständlich nicht limitierend sein.

Es kann auch von Vorteil sein, UVA-Filter einzusetzen, die üblicherweise in kosmetischen Zubereitungen enthalten sind. Bei diesen Substanzen handelt es sich vorzugsweise um Derivate des Dibenzoylmethans, insbesondere um 1-(4'-tert.Butylphenyl)-3-(4'-methoxyphenyl)-propan-1,3-dion und um 1-Phenyl-3-(4'-isopropylphenyl)propan-1,3-dion. Es können die für die UVB-Kombination verwendeten Mengen eingesetzt werden.

Erfindungsgemäß enthalten kosmetische und dermatologische Zubereitungen vorteilhaft außerdem anorganische Pigmente auf Basis von Metalloxiden und/oder anderen in Wasser schwerlöslichen oder unlöslichen Metallverbindungen, insbesondere der Oxide des Titans (TiO₂), Zinks (ZnO), Eisens (z.B. Fe₂O₃), Zirkoniums (ZrO₂), Siliciums (SiO₂), Mangans (z.B. MnO), Aluminiums (Al₂O₃), Cers (z.B. Ce₂O₃), Mischoxiden der entsprechenden Metalle sowie Abmischungen aus solchen Oxiden. Besonders bevorzugt handelt es sich um Pigmente auf der Basis von TiO₂.

Es ist besonders vorteilhaft im Sinne der vorliegenden Erfindung, wenngleich nicht zwingend, wenn die anorganischen Pigmente in hydrophober Form vorliegen, d.h., dass sie oberflächlich wasserabweisend behandelt sind. Diese Oberflächenbehandlung kann darin bestehen, dass die Pigmente nach an sich bekannten Verfahren mit einer dünnen hydrophoben Schicht versehen werden.

Eines solcher Verfahren besteht beispielsweise darin, dass die hydrophobe Oberflächenschicht nach einer Reaktion gemäß

nTiO₂ + m(RO)₃Si-R' -> nTiO₂ (oberfl.)

erzeugt wird. n und m sind dabei nach Belieben einzusetzende stöchiometrische Parameter, R und R' die gewünschten organischen Reste. Beispielsweise in Analogie zu DE-OS 33 14 742 dargestellte hydrophobisierte Pigmente sind von Vorteil.

Vorteilhafte TiO₂-Pigmente sind beispielsweise unter den Handelsbezeichnungen MT 100 T von der Firma TAYCA, ferner M 160 von der Firma Kemira sowie T 805 von der Firma Degussa erhältlich.

Zubereitungen gemäß der Erfindung können, zumal wenn kristalline oder mikrokristalline Festkörper, beispielsweise anorganische Mikropigmente in die erfindungsgemäßen Zubereitungen eingearbeitet werden sollen, auch anionische, nichtionische und/oder amphotere Tenside enthalten. Tenside sind amphiphile Stoffe, die organische, unpolare Substanzen in Wasser lösen können.

Bei den hydrophilen Anteilen eines Tensidmoleküls handelt es sich meist um polare funktionelle Gruppen, beispielweise -COO⁻, -OSO₃²⁻, -SO₃⁻, während die hydrophoben Teile in der Regel unpolare Kohlenwasserstoffreste darstellen. Tenside werden im Allgemeinen nach Art und Ladung des hydrophilen Molekülteils klassifiziert. Hierbei können vier Gruppen unterschieden werden:
- anionische Tenside,
- kationische Tenside,
- amphotere Tenside und
- nichtionische Tenside.

Anionische Tenside weisen als funktionelle Gruppen in der Regel Carboxylat-, Sulfat- oder Sulfonatgruppen auf. In wässriger Lösung bilden sie im sauren oder neutralen Milieu negativ geladene organische Ionen. Kationische Tenside sind beinahe ausschließlich durch das Vorhandensein einer quaternären Ammoniumgruppe gekennzeichnet. In wässriger Lösung bilden sie im sauren oder neutralen Milieu positiv geladene organische Ionen. Amphotere Tenside enthalten sowohl anionische als auch kationische Gruppen und verhalten sich demnach in wässriger Lösung je nach pH-Wert wie anionische oder kationische Tenside. Im stark sauren Milieu besitzen sie eine positive und im alkalischen Milieu eine negative Ladung. Im neutralen pH-Bereich hingegen sind sie zwitterionisch, wie das folgende Beispiel verdeutlichen soll:

| | | |
|---|---|---|
| RNH₂⁺CH₂CH₂COOH X⁻ | (bei pH=2) | X⁻ = beliebiges Anion, z.B. Cl⁻ |
| RNH₂⁺CH₂CH₂COO⁻ | (bei pH=7) | |
| RNHCH₂CH₂COO⁻ B⁺ | (bei pH=12) | B⁺ = beliebiges Kation, z.B. Na⁺ |

Typisch für nicht-ionische Tenside sind Polyether-Ketten. Nicht-ionische Tenside bilden in wässrigem Medium keine Ionen.

### A. Anionische Tenside

### Vorteilhaft zu verwendende anionische Tenside sind

Acylaminosäuren (und deren Salze), wie
1. Acylglutamate, beispielsweise Natriumacylglutamat, Di-TEA-palmitoylaspartat und Natrium Caprylic/ Capric Glutamat,
2. Acylpeptide, beispielsweise Palmitoyl-hydrolysiertes Milchprotein, Natrium Cocoyl-hydrolysiertes Soja Protein und Natrium-/ Kalium Cocoyl-hydrolysiertes Kollagen,
3. Sarcosinate, beispielsweise Myristoyl Sarcosin, TEA-lauroyl Sarcosinat, Natriumlauroylsarcosinat und Natriumcocoylsarkosinat,
4. Taurate, beispielsweise Natriumlauroyltaurat und Natriummethylcocoyltaurat,
5. Acyllactylate, Lauroyllactylat, Caproyllactylat,
6. Alaninate.

Carbonsäuren und Derivate, wie
1. Carbonsäuren, beispielsweise Laurinsäure, Aluminiumstearat, Magnesiumalkanolat und Zinkundecylenat,
2. Ester-Carbonsäuren, beispielsweise Calciumstearoyllactylat, Laureth-6 Citrat und Natrium PEG-4 Lauramidcarboxylat,
3. Ether-Carbonsäuren, beispielsweise Natriumlaureth-13 Carboxylat und Natrium PEG-6 Cocamide Carboxylat,

Phosphorsäureester und Salze, wie beispielsweise DEA-Oleth-10-Phosphat und Dilaureth-4 Phosphat,
Sulfonsäuren und Salze, wie
1. Acyl-isethionate, z.B. Natrium-/ Ammoniumcocoyl-isethionat,
2. Alkylarylsulfonate,
3. Alkylsulfonate, beispielsweise Natriumcocosmonoglyceridsulfat, Natrium C₁₂₋₁₄ Olefinsulfonat, Natriumlaurylsulfoacetat und Magnesium PEG-3 Cocamidsulfat,
4. Sulfosuccinate, beispielsweise Dioctylnatriumsulfosuccinat, Dinatriumlaurethsulfosuccinat, Dinatriumlaurylsulfosuccinat und Dinatriumundecylenamido MEA-Sulfosuccinat
sowie

Schwefelsäureester, wie
1. Alkylethersulfat, beispielsweise Natrium-, Ammonium-, Magnesium-, MIPA-, TIPA- Laurethsulfat, Natriummyrethsulfat und Natrium C₁₂₋₁₃ Parethsulfat,
2. Alkylsulfate, beispielsweise Natrium-, Ammonium- und TEA- Laurylsulfat.

### B. Kationische Tenside

Vorteilhaft zu verwendende kationische Tenside sind
1. Alkylamine,
2. Alkylimidazole,
3. Ethoxylierte Amine,
4. Quaternäre Tenside,
5. Esterquats.

Quaternäre Tenside enthalten mindestens ein N-Atom, das mit 4 Alkyl- oder Arylgruppen kovalent verbunden ist. Dies führt, unabhängig vom pH Wert, zu einer positiven Ladung. Vorteilhaft sind, Alkylbetain, Alkylamidopropylbetain und Alkyl-amidopropylhydroxysulfain. Die erfindungsgemäß verwendeten kationischen Tenside können ferner bevorzugt gewählt werden aus der Gruppe der quaternären Ammoniumverbindungen, insbesondere Benzyltrialkylammoniumchloride oder -bromide, wie beispielsweise Benzyldimethylstearylammoniumchlorid, ferner Alkyltrialkylammoniumsalze, beispielsweise beispielsweise Cetyltrimethylammoniumchlorid oder -bromid, Alkyldimethylhydroxyethylammoniumchloride oder -bromide, Dialkyldimethylammoniumchloride oder -bromide, Alkylamidethyltrimethylammoniumethersulfate, Alkylpyridiniumsalze, beispielsweise Lauryl- oder Cetylpyrimidiniumchlorid, Imidazolinderivate und Verbindungen mit kationischem Charakter wie Aminoxide, beispielsweise Alkyldimethylaminoxide oder Alkylaminoethyldimethylaminoxide. Vorteilhaft sind insbesondere Cetyltrimethylammoniumsalze zu verwenden.

### C. Amphotere Tenside

Vorteilhaft zu verwendende amphotere Tenside sind
1. Acyl-/dialkylethylendiamin, beispielsweise Natriumacylamphoacetat, Dinatriumacylamphodipropionat, Dinatriumalkylamphodiacetat, Natriumacylamphohydroxypropylsulfonat, Dinatriumacylamphodiacetat und Natriumacylamphopropionat,
2. N-Alkylaminosäuren, beispielsweise Aminopropylalkylglutamid, Alkylaminopropionsäure, Natriumalkylimidodipropionat und Lauroamphocarboxyglycinat.

### D. Nicht-ionische Tenside

Vorteilhaft zu verwendende nicht-ionische Tenside sind
1. Alkohole,
2. Alkanolamide, wie Cocamide MEA/ DEA/ MIPA,
3. Aminoxide, wie Cocoamidopropylaminoxid,
4. Ester, die durch Veresterung von Carbonsäuren mit Ethylenoxid, Glycerin, Sorbitan oder anderen Alkoholen entstehen,
5. Ether, beispielsweise ethoxylierte/propoxylierte Alkohole, ethoxylierte/ propoxylierte Ester, ethoxylierte/ propoxylierte Glycerinester, ethoxylierte/ propoxylierte Cholesterine, ethoxylierte/ propoxylierte Triglyceridester, ethoxyliertes propoxyliertes Lanolin, ethoxylierte/ propoxylierte Polysiloxane, propoxylierte POE-Ether und Alkylpolyglycoside wie Laurylglucosid, Decylglycosid und Cocoglycosid,
6. Sucroseester, -Ether,
7. Polyglycerinester, Diglycerinester, Monoglycerinester,
8. Methylglucosester, Ester von Hydroxysäuren.

Vorteilhaft ist ferner die Verwendung einer Kombination von anionischen und/oder amphoteren Tensiden mit einem oder mehreren nicht-ionischen Tensiden.

Die oberflächenaktive Substanz kann in einer Konzentration zwischen 1 und 95 Gew.-% in den erfindungsgemäßen Zubereitungen vorliegen, bezogen auf das Gesamtgewicht der Zubereitungen.

Zubereitungen gemäß der Erfindung können auch in Form kosmetischer Desodorantien und/oder Antitranspirantien vorliegen. Desodorantien sollen folgende Bedingungen erfüllen:
1) Sie sollen eine zuverlässige Desodorierung bewirken.
2) Die natürlichen biologischen Vorgänge der Haut dürfen nicht durch die Desodorantien beeinträchtigt werden.
3) Die Desodorantien müssen bei Überdosierung oder sonstiger nicht bestimmungsgemäßer Anwendung unschädlich sein.
4) Sie sollen sich nach wiederholter Anwendung nicht auf der Haut anreichern.
5) Sie sollen sich gut in übliche kosmetische Formulierungen einarbeiten lassen.

Bekannt und gebräuchlich sind sowohl flüssige Desodorantien, beispielsweise Aerosolsprays, Roll-ons und dergleichen als auch feste Zubereitungen, beispielsweise Deo-Stifte ("Sticks"), Puder, Pudersprays, Intimreinigungsmittel usw.

In sogenannten Antitranspirantien kann durch Adstringentien - vorwiegend Aluminiumsalze wie Aluminiumhydroxychlorid (Aluchlorhydrat) - die Bildung des Schweißes reduziert werden.

Durch die Verwendung antimikrobieller Stoffe in kosmetischen Desodorantien kann die Bakterienflora auf der Haut reduziert werden. Dabei sollten im Idealfalle nur die Geruch verursachenden Mikroorganismen wirksam reduziert werden. Der Schweißfluss selbst wird dadurch nicht beeinflusst, im Idealfalle wird nur die mikrobielle Zersetzung des Schweißes zeitweilig gestoppt.

Auch die Kombination von Adstringentien mit antimikrobiell wirksamen Stoffen in ein und derselben Zusammensetzung ist gebräuchlich.

Bekannt und gebräuchlich sind neben den flüssigen Desodorantien auch feste Zubereitungen, beispielsweise Deo-Stifte ("Sticks"), Puder, Pudersprays, Intimreinigungsmittel usw.

Die Lipidphase der erfindungsgemäßen kosmetischen oder dermatologischen Emulsionen kann vorteilhaft gewählt werden aus folgender Substanzgruppe:
- Mineralöle, Mineralwachse
- Öle, wie Triglyceride der Caprin- oder der Caprylsäure, ferner natürliche Öle wie z.B. Rizinusöl;
- Fette, Wachse und andere natürliche und synthetische Fettkörper, vorzugsweise Ester von Fettsäuren mit Alkoholen niedriger C-Zahl, z.B. mit Isopropanol, Propylenglykol oder Glycerin, oder Ester von Fettalkoholen mit Alkansäuren niedriger C-Zahl oder mit Fettsäuren;
- Alkylbenzoate;
- Silikonöle wie Dimethylpolysiloxane, Diethylpolysiloxane, Diphenylpolysiloxane sowie Mischformen daraus.

Die Ölphase der Emulsionen gemäß der vorliegenden Erfindung wird vorteilhaft gewählt aus der Gruppe der Ester aus gesättigten und/oder ungesättigten, verzweigten und/oder unverzweigten Alkancarbonsäuren einer Kettenlänge von 3 bis 30 C-Atomen und gesättigten und/oder ungesättigten, verzweigten und/oder unverzweigten Alkoholen einer Kettenlänge von 3 bis 30 C-Atomen, aus der Gruppe der Ester aus aromatischen Carbonsäuren und gesättigten und/oder ungesättigten, verzweigten und/oder unverzweigten Alkoholen einer Kettenlänge von 3 bis 30 C-Atomen. Solche Esteröle können dann vorteilhaft gewählt werden aus der Gruppe Isopropylmyristat, Isopropylpalmitat, Isopropylstearat, Isopropyloleat, n-Butylstearat, n-Hexyllaurat, n-Decyloleat, Isooctylstearat, Isononylstearat, Isononylisononanoat, 2-Ethylhexylpalmitat, 2-Ethylhexyllaurat, 2-Hexyldecylstearat, 2-Octyldodecylpalmitat, Oleyloleat, Oleylerucat, Erucyloleat, Erucylerucat sowie synthetische, halbsynthetische und natürliche Gemische solcher Ester, z.B. Jojobaöl.

Ferner kann die Ölphase vorteilhaft gewählt werden aus der Gruppe der verzweigten und unverzweigten Kohlenwasserstoffe und -wachse, der Silkonöle, der Dialkylether, der Gruppe der gesättigten oder ungesättigten, verzweigten oder unverzweigten Alkohole, sowie der Fettsäuretriglyceride, namentlich der Triglycerinester gesättigter und/oder ungesättigter, verzweigter und/oder unverzweigter Alkancarbonsäuren einer Kettenlänge von 8 bis 24, insbesondere 12 - 18 C-Atomen. Die Fettsäuretriglyceride können beispielsweise vorteilhaft gewählt werden aus der Gruppe der synthetischen, halbsynthetischen und natürlichen Öle, z.B. Olivenöl, Sonnenblumenöl, Sojaöl, Erdnussöl, Rapsöl, Mandelöl, Palmöl, Kokosöl, Palmkernöl und dergleichen mehr.

Auch beliebige Abmischungen solcher Öl- und Wachskomponenten sind vorteilhaft im Sinne der vorliegenden Erfindung einzusetzen. Es kann auch gegebenenfalls vorteilhaft sein, Wachse, beispielsweise Cetylpalmitat, als alleinige Lipidkomponente der Ölphase einzusetzen.

Vorteilhaft wird die Ölphase gewählt aus der Gruppe 2-Ethylhexylisostearat, Octyldodecanol, Isotridecylisononanoat, Isoeicosan, 2-Ethylhexylcocoat, C₁₂₋₁₅-Alkylbenzoat, Capryl-Caprinsäure-triglycerid, Dicaprylylether.

Besonders vorteilhaft sind Mischungen aus C₁₂₋₁₅-Alkylbenzoat und 2-Ethylhexylisostearat, Mischungen aus C₁₂₋₁₅-Alkylbenzoat und Isotridecylisononanoat sowie Mischungen aus C₁₂₋₁₅-Alkylbenzoat, 2-Ethylhexylisostearat und Isotridecylisononanoat.

Von den Kohlenwasserstoffen sind Paraffinöl, Squalan und Squalen vorteilhaft im Sinne der vorliegenden Erfindung zu verwenden.

Vorteilhaft kann die Ölphase ferner einen Gehalt an cyclischen oder linearen Silikonölen aufweisen oder vollständig aus solchen Ölen bestehen, wobei allerdings bevorzugt wird, außer dem Silikonöl oder den Silikonölen einen zusätzlichen Gehalt an anderen Ölphasenkomponenten zu verwenden. Solche Silicone oder Siliconöle können als Monomere vorliegen, welche in der Regel durch Strukturelemente charakterisiert sind, wie folgt:

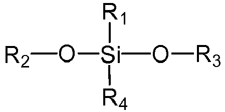

Als erfindungsgemäß vorteilhaft einzusetzende lineare Silicone mit mehreren Siloxyleinheiten werden im allgemeinen durch Strukturelemente charakterisiert wie folgt: 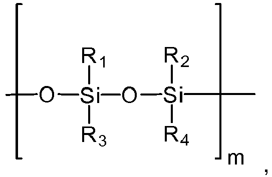 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). m kann dabei Werte von 2 - 200.000 annehmen.

Erfindungsgemäß vorteilhaft einzusetzende cyclische Silicone werden im allgemeinen durch Strukturelemente charakterisiert, wie folgt 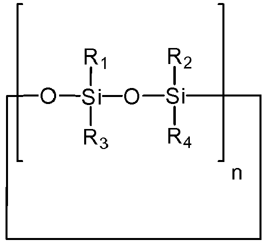 wobei die Siliciumatome mit gleichen oder unterschiedlichen Alkylresten und/oder Arylresten substituiert werden können, welche hier verallgemeinernd durch die Reste R₁ - R₄ dargestellt sind (will sagen, dass die Anzahl der unterschiedlichen Reste nicht notwendig auf bis zu 4 beschränkt ist). n kann dabei Werte von 3/2 bis 20 annehmen. Gebrochene Werte für n berücksichtigen, dass ungeradzahlige Anzahlen von Siloxylgruppen im Cyclus vorhanden sein können.

Vorteilhaft wird Cyclomethicon (z.B. Decamethylcyclopentasiloxan) als erfindungsgemäß zu verwendendes Silikonöl eingesetzt. Aber auch andere Silikonöle sind vorteilhaft im Sinne der vorliegenden Erfindung zu verwenden, beispielsweise Polydimethylsiloxan, Poly(methylphenylsiloxan), Cetyldimethicon, Behenoxydimethicon.

Es ist aber auch vorteilhaft, Silikonöle ähnlicher Konstitution wie der vorstehend bezeichneten Verbindungen zu wählen, deren organische Seitenketten derivatisiert, beispielsweise polyethoxyliert und/oder polypropoxyliert sind. Dazu zählen beispielsweise Polysiloxan-polyalkyl-polyether-copolymere wie das Cetyl-Dimethicon-Copolyol, das (Cetyl-Dimethicon-Copolyol (und) Polyglyceryl-4-Isostearat (und) Hexyllaurat).

Besonders vorteilhaft sind ferner Mischungen aus Cyclomethicon und Isotridecylisononanoat, aus Cyclomethicon und 2-Ethylhexylisostearat.

Die wässriger Phase der Zubereitungen gemäß der Erfindung enthält gegebenenfalls vorteilhaft Alkohole, Diole oder Polyole niedriger C-Zahl, sowie deren Ether, vorzugsweise Ethanol, Isopropanol, Propylenglykol, Glycerin, Ethylenglykol, Ethylenglykolmonoethyl- oder-monobutylether, Propylenglykolmonomethyl, -monoethyl- oder -monobutylether, Diethylenglykolmonomethyl- oder -monoethylether und analoge Produkte, ferner Alkohole niedriger C-Zahl, z.B. Ethanol, Isopropanol, 1,2-Propandiol, Glycerin sowie insbesondere ein oder mehrere Verdickungsmittel, welches oder welche vorteilhaft gewählt werden können aus der Gruppe Siliciumdioxid, Aluminiumsilikate.

Erfindungsgemäß enthalten als Emulsionen vorliegenden Zubereitungen insbesondere vorteilhaft ein oder mehrere Hydrocolloide. Diese Hydrocolloide können vorteilhaft gewählt werden aus der Gruppe der Gummen, Polysaccharide, Cellulosederivate, Schichtsilikate, Polyacrylate und/oder anderen Polymeren.

Erfindungsgemäß enthalten als Hydrogele vorliegenden Zubereitungen ein oder mehrere Hydrocolloide. Diese Hydrocolloide können vorteilhaft aus der vorgenannten Gruppe gewählt werden.

Zu den Gummen zählt man Pflanzen- oder Baumsäfte, die an der Luft erhärten und Harze bilden oder Extrakte aus Wasserpflanzen. Aus dieser Gruppe können vorteilhaft im Sinne der vorliegenden Erfindung gewählt werden beispielsweise Gummi Arabicum, Johannisbrotmehl, Tragacanth, Karaya, Guar Gummi, Pektin, Gellan Gummi, Carrageen, Agar, Algine, Chondrus, Xanthan Gummi.

Weiterhin vorteilhaft ist die Verwendung von derivatisierten Gummen wie z.B. Hydroxypropyl Guar (Jaguar® HP 8).

Unter den Polysacchariden und -derivaten befinden sich z.B. Hyaluronsäure, Chitin und Chitosan, Chondroitinsulfate, Stärke und Stärkederivate.

Unter den Cellulosederivaten befinden sich z.B. Methylcellulose, Carboxymethylcellulose, Hydroxyethylcellulose, Hydroxypropylmethylcellulose.

Unter den Schichtsilikaten befinden sich natürlich vorkommende und synthetische Tonerden wie z.B. Montmorillonit, Bentonit, Hektorit, Laponit, Magnesiumaluminiumsilikate wie Veegum®. Diese können als solche oder in modifizierter Form verwendet werden wie z.B. Stearylalkonium Hektorite.

Weiterhin können vorteilhaft auch Kieselsäuregele verwendet werden.

Unter den Polyacrylaten befinden sich z.B. Carbopol Typen der Firma Lubrizol (Carbopol 980, 981, 1382, 5984, 2984, EDT 2001 oder Pemulen TR2).

Unter den Polymeren befinden sich z.B. Polyacrylamide (Sepigel 305), Polyvinylalkohole, PVP, PVP / VA Copolymere, Polyglycole.

Erfindungsgemäß enthalten als Emulsionen vorliegenden Zubereitungen einen oder mehrere anionische Emulgatoren.

O/W-Emulgatoren können beispielsweise vorteilhaft gewählt werden aus der Gruppe der polyethoxylierten bzw. polypropoxylierten bzw. polyethoxylierten und polypropoxylierten Produkte, z.B.:
- der Fettalkoholethoxylate
- der ethoxylierten Wollwachsalkohole,
- der Polyethylenglycolether der allgemeinen Formel R-O-(-CH₂-CH₂-O-)ₙ-R',
- der Fettsäureethoxylate der allgemeinen Formel

   R-COO-(-CH₂-CH₂-O-)ₙ-H,
- der veretherten Fettsäureethoxylate der allgemeinen Formel

   R-COO-(-CH₂-CH₂-O-)ₙ-R',
- der veresterten Fettsäureethoxylate der allgemeinen Formel

   R-COO-(-CH₂-CH₂-O-)ₙ -C(O)-R',
- der Polyethylenglycolglycerinfettsäureester
- der ethoxylierten Sorbitanester
- der Cholesterinethoxylate
- der ethoxylierten Triglyceride
- der Alkylethercarbonsäuren der allgemeinen Formel R-O-(-CH₂-CH₂-O-)ₙ-CH₂-COOH und n eine Zahl von 5 bis 30 darstellen,
- der Polyoxyethylensorbitolfettsäureester,
- der Alkylethersulfate der allgemeinen Formel R-O-(-CH₂-CH₂-O-)ₙ-SO₃-H
- der Fettalkoholpropoxylate der allgemeinen Formel

   R-O-(-CH₂-CH(CH₃)-O-)ₙ-H,
- der Polypropylenglycolether der allgemeinen Formel

   R-O-(-CH₂-CH(CH₃)-O-)ₙ-R',
- der propoxylierten Wollwachsalkohole,
- der veretherten Fettsäurepropoxylate

   R-COO-(-CH₂-CH(CH₃)-O-)ₙ-R',
- der veresterten Fettsäurepropoxylate der allgemeinen Formel

   R-COO-(-CH₂-CH(CH₃)-O-)ₙ-C(O)-R',
- der Fettsäurepropoxylate der allgemeinen Formel

   R-COO-(-CH₂-CH(CH₃)-O-)ₙ-H,
- der Polypropylenglycolglycerinfettsäureester
- der propoxylierten Sorbitanester
- der Cholesterinpropoxylate
- der propoxylierten Triglyceride
- der Alkylethercarbonsäuren der allgemeinen Formel

   R-O-(-CH₂-CH(CH₃)O-)ₙ-CH₂-COOH
- der Alkylethersulfate bzw. die diesen Sulfaten zugrundeliegenden Säuren der allgemeinen Formel R-O-(-CH₂-CH(CH₃)-O-)ₙ-SO₃-H
- der Fettalkoholethoxylate/propoxylate der allgemeinen Formel

   R-O-Xₙ-Yₘ-H,
- der Polypropylenglycolether der allgemeinen Formel

   R-O-Xₙ-Yₘ-R',
- der veretherten Fettsäurepropoxylate der allgemeinen Formel

   R-COO-Xₙ-Yₘ-R',
- der Fettsäureethoxylate/propoxylate der allgemeinen Formel

   R-COO-Xₙ-Yₘ-H.

Erfindungsgemäß besonders vorteilhaft werden die eingesetzten polyethoxylierten bzw. polypropoxylierten bzw. polyethoxylierten und polypropoxylierten O/W-Emulgatoren gewählt aus der Gruppe der Substanzen mit HLB-Werten von 11 - 18, ganz besonders vorteilhaft mit mit HLB-Werten von 14,5 - 15,5, sofern die O/W-Emulgatoren gesättigte Reste R und R' aufweisen. Weisen die O/W-Emulgatoren ungesättigte Reste R und/oder R' auf, oder liegen Isoalkylderivate vor, so kann der bevorzugte HLB-Wert solcher Emulgatoren auch niedriger oder darüber liegen.

Es ist von Vorteil, die Fettalkoholethoxylate aus der Gruppe der ethoxylierten Stearylalkohole, Cetylalkohole, Cetylstearylalkohole (Cetearylalkohole) zu wählen. Insbesondere bevorzugt sind:
Polyethylenglycol(13)stearylether (Steareth-13), Polyethylenglycol(14)stearylether (Steareth-14), Polyethylenglycol(15)stearylether (Steareth-15), Polyethylenglycol(16)stearylether (Steareth-16), Polyethylenglycol(17)stearylether (Steareth-17), Polyethylenglycol(18)stearylether (Steareth-18), Polyethylenglycol(19)stearylether (Steareth-19), Polyethylenglycol(20)stearylether(Steareth-20),
Polyethylenglycol(12)isostearylether (Isosteareth-12), Polyethylenglycol(13)isostearylether (Isosteareth-13), Polyethylenglycol(14)isostearylether (Isosteareth-14), Polyethylenglycol-(15)isostearylether (Isosteareth-15), Polyethylenglycol(16)isostearylether (Isosteareth-16), Polyethylenglycol(17)isostearylether (Isosteareth-17), Polyethylenglycol(18)isostearylether (Isosteareth-18), Polyethylenglycol(19)isostearylether (Isosteareth-19), Polyethylenglycol-(20)isostearylether (Isosteareth-20),
Polyethylenglycol(13)cetylether (Ceteth-13), Polyethylenglycol(14)cetylether (Ceteth-14), Polyethylenglycol(15)cetylether (Ceteth-15), Polyethylenglycol(16)cetylether (Ceteth-16), Polyethylenglycol(17)cetylether (Ceteth-17), Polyethylenglycol(18)cetylether (Ceteth-18), Polyethylenglycol(19)cetylether (Ceteth-19), Polyethylenglycol(20)cetylether (Ceteth-20),
Polyethylenglycol(13)isocetylether (Isoceteth-13), Polyethylenglycol(14)isocetylether (Isoceteth-14), Polyethylenglycol(15)isocetylether (Isoceteth-15), Polyethylenglycol(16)isocetylether (Isoceteth-16), Polyethylenglycol(17)isocetylether (Isoceteth-17), Polyethylenglycol-(18)isocetylether (Isoceteth-18), Polyethylenglycol(19)isocetylether (Isoceteth-19), Polyethylenglycol(20)isocetylether (Isoceteth-20),
Polyethylenglycol(12)oleylether (Oleth-12), Polyethylenglycol(13)oleylether (Oleth-13), Polyethylenglycol(14)oleylether (Oleth-14), Polyethylenglycol(15)oleylether (Oleth-15),
Polyethylenglycol(12)laurylether (Laureth-12), Polyethylenglycol(12)isolaurylether (Isolaureth-12).

Polyethylenglycol(13)cetylstearylether (Ceteareth-13), Polyethylenglycol(14)cetylstearylether (Ceteareth-14), Polyethylenglycol(15)cetylstearylether (Ceteareth-15), Polyethylenglycol(16)-cetylstearylether (Ceteareth-16), Polyethylenglycol(17)cetylstearylether (Ceteareth-17), Polyethylenglycol(18)cetylstearylether (Ceteareth-18), Polyethylenglycol(19)cetylstearylether (Ceteareth-19), Polyethylenglycol(20)cetylstearylether (Ceteareth-20),
Es ist ferner von Vorteil, die Fettsäureethoxylate aus folgender Gruppe zu wählen:
Polyethylenglycol(20)stearat, Polyethylenglycol(21)stearat, Polyethylenglycol(22)stearat, Polyethylenglycol(23)stearat, Polyethylenglycol(24)stearat, Polyethylenglycol(25)stearat,
Polyethylenglycol(12)isostearat, Polyethylenglycol(13)isostearat, Polyethylenglycol(14)isostearat, Polyethylenglycol(15)isostearat, Polyethylenglycol(16)isostearat, Polyethylenglycol-(17)isostearat, Polyethylenglycol(18)isostearat, Polyethylenglycol(19)isostearat, Polyethylenglycol(20)isostearat, Polyethylenglycol(21)isostearat, Polyethylenglycol(22)isostearat, Polyethylenglycol(23)isostearat, Polyethylenglycol(24)isostearat, Polyethylenglycol(25)isostearat,
Polyethylenglycol(12)oleat, Polyethylenglycol(13)oleat, Polyethylenglycol(14)oleat, Polyethylenglycol(15)oleat, Polyethylenglycol(16)oleat, Polyethylenglycol(17)oleat, Polyethylenglycol-(18)oleat, Polyethylenglycol(19)oleat, Polyethylenglycol(20)oleat

Als ethoxylierte Alkylethercarbonsäure bzw. deren Salz kann vorteilhaft das Natriumlaureth-11-carboxylat verwendet werden.

Als Alkylethersulfat kann Natrium Laureth 1-4 sulfat vorteilhaft verwendet werden.

Als ethoxyliertes Cholesterinderivat kann vorteilhaft Polyethylenglycol(30)Cholesterylether verwendet werden. Auch Polyethylenglycol(25)Sojasterol hat sich bewährt.

Als ethoxylierte Triglyceride können vorteilhaft die Polyethylenglycol(60) Evening Primrose Glycerides verwendet werden (Evening Primrose = Nachtkerze)

Weiterhin ist von Vorteil, die Polyethylenglycolglycerinfettsäureester aus der Gruppe Polyethylenglycol(20)glyceryllaurat, Polyethylenglycol(21)glyceryllaurat, Polyethylenglycol(22)glyceryllaurat, Polyethylenglycol(23)glyceryllaurat, Polyethylenglycol(6)glycerylcaprat/caprinat, Polyethylenglycol(20)glyceryloleat, Polyethylenglycol(20)glycerylisostearat, Polyethylenglycol(18)glyceryloleat/cocoat zu wählen.

Es ist ebenfalls günstig, die Sorbitanester aus der Gruppe Polyethylenglycol(20)sorbitanmonolaurat, Polyethylenglycol(20)sorbitanmonostearat, Polyethylenglycol(20)sorbitanmonoisostearat, Polyethylenglycol(20)sorbitanmonopalmitat, Polyethylenglycol(20)sorbitanmonooleat zu wählen.

Als vorteilhafte W/O-Emulgatoren können eingesetzt werden: Fettalkohole mit 8 bis 30 Kohlenstoffatomen, Monoglycerinester gesättigter und/oder ungesättigter, verzweigter und/oder unverzweigter Alkancarbonsäuren einer Kettenlänge von 8 bis 24, insbesondere 12 - 18 C-Atomen, Diglycerinester gesättigter und/oder ungesättigter, verzweigter und/oder unverzweigter Alkancarbonsäuren einer Kettenlänge von 8 bis 24, insbesondere 12 - 18 C-Atomen, Monoglycerinether gesättigter und/oder ungesättigter, verzweigter und/oder unverzweigter Alkohole einer Kettenlänge von 8 bis 24, insbesondere 12 - 18 C-Atomen, Diglycerinether gesättigter und/oder ungesättigter, verzweigter und/oder unverzweigter Alkohole einer Kettenlänge von 8 bis 24, insbesondere 12 - 18 C-Atomen, Propylenglycolester gesättigter und/oder ungesättigter, verzweigter und/oder unverzweigter Alkancarbonsäuren einer Kettenlänge von 8 bis 24, insbesondere 12 - 18 C-Atomen sowie Sorbitanester gesättigter und/oder ungesättigter, verzweigter und/oder unverzweigter Alkancarbonsäuren einer Kettenlänge von 8 bis 24, insbesondere 12 - 18 C-Atomen.

Liegt in den erfindungsgemäßen Ausführungsformen der vorliegenden Erfindung die Emulsion in Form einer W/O-Emulsion vor, so ist es erfindungsgemäß vorteilhaft einen oder mehrere W/O-Emulgatoren aus der Gruppe der Verbindungen Polyglycerylisostearate, ethoxylierten Stearate, ethoxylierten hydrierten Rizinusöle, Alkylisostearate, ethoxylierte Dodeceylglycolcopolymere und Wollwachsderivate zu wählen.

Dabei ist der Einsatz der W/O-Emulgatoren Polyglyceryl-3 Diiosostearat und PEG-30 Dipolyhydroxystearat erfindungsgemäß bevorzugt.

Liegt in den erfindungsgemäßen Ausführungsformen der vorliegenden Erfindung die Emulsion in Form einer W/S-Emulsion vor, so ist es erfindungsgemäß vorteilhaft einen oder mehrere W/S-Emulgatoren aus der Gruppe der Verbindungen ethoxylierten-proxylierten alkylmodifizierten Dimethicone und Dimethicone Copolyole zu wählen.

Dabei ist der Einsatz der W/S-Emulgatoren Dimethiconcopolyol und Cetyl PEG/PPG-10/1 Dimethicone erfindungsgemäß bevorzugt.

Es hat sich im Übrigen in überraschender Weise herausgestellt, dass der Dufteindruck einer kosmetischen Zubereitung durch die Zugabe von 4-Hydroxyacetophenon, gegenüber einer solchen Zubereitung, die kein 4-Hydroxyacetophenon enthält, deutlich verbessert wird.

Dies ist umso überraschender, als insbesondere 4-Hydroxyacetophenon und ihre Analoga und insbesondere 4-Hydroxyacetophenon-haltige Hautpflegeprodukte einen Nebengeruch ausbilden können, der für den Anwender als unangenehm empfunden wird.

Gerade wenn 4-Hydroxyacetophenon als Bestandteil einer Körperlotion formuliert worden ist und die ersten leichtflüchtigen Parfumbestandteile verdampft sind, kann es zu einem Nebengeruch kommen, der 4-Hydroxyacetophenon zuzuschreiben ist.

Als vorteilhafte Verkörperung der vorliegenden Erfindung werden daher auch Stoffkombinationen aus 4-Hydroxyacetophenon und einem oder mehreren Parfumstoffen angesehen.

Als vorteilhafte Parfumstoffe können eingesetzt werden: Dipropylenglycol, Methyl dihydrojasmonate, Phenethylalcohol, Linalool, Linalyl acetate, 2,6-Dimethyl-7-octen-2-ol, alpha-Hexylcinnamaldehyd, 2-Acetonapthone-1,2,3,4,5,6,7,8-octahydro-2,3,8,8-tetramethyl, p-t-Butylalpha-methyldihydrocinnamic aldehyde, Benzyl acetate, 1,3,4,6,7,8-Hexahydro-4,6,6,7,8,8-hexamethylcyclopenta-gamma-2-benzopyran, Methyl cedryl ketone, Ethylene brassylate, 4-(4-Hydroxy-4-methylpentyl)-3-cyclohexene-1-carboxyaldehyde, Benzyl salicylate, Hexyl salicylate, Orange oil, alpha-Isomethylionone, Diethyl phthalate, 4-t-Butylcyclohexyl acetate, Patchouli oil, 3,7-Dimethyl-2,6-octadien-1-ol, Tetrahydrolinalool, Hydroxycitronellal, Isopropyl myristate, 3,7-Dimethyl-6-octen-1-ol, Orange terpenes, Heliotropin, Terpinyl acetate, omega-Pentadecalactone, Methyl-alpha-ionone, Lavandin oil, Lemon oil, Bergamot oil, 7-Acetyl-1,1,3,4,4,6-hexamethyltetralin, Coumarin, Ethyllinalool, Amyl salicylate, 2-tert-Pentyl-cyclohexyl acetate, 3-Methyl-5-phenyl-1-pentanol, Cedrol, Benzyl benzoate, Vanillin, alpha-Amylcinnamaldehyde, Dimethyl phthalate, d-Limonene, 2-Isobutyl-4-hydroxy-4-methyltetrahydropyran, Triethyl citrate, Terpineol, Lavender oil, Diethylene glycol monoethyl ether, 2-Phenoxyethyl isobutyrate, Anisyl alcohol, 3-Pentyltetrahydro(2H)pyranyl acetate, Methyl ester of rosin, partially hydrogenated, Isobornyl acetate, Rosemary oil, Petitgrain oil, 1,4-Dioxacyclohexadecane-5,16-dione, Isoamyl salicylate, gamma-Undecalactone, alpha-Ionone, Oxacyclohexadecen-2-one, 7-Octen-2-ol, 2-methyl-6-methylene, dihydro derive, 1,2-Propyleneglycol, 3-(5,5,6-Trimethylbicyclo(2.2.1)hept-2-yl)cyclohexan-1-ol, Geranium oil, Musk ketone, Cedrenyl acetate, Isobornylcyclohexanol, Ionone, Benzyl alcohol, gamma-Nonalactone, I-Menthol, Cyclohexyl salicylate, Dihydromyrcenyl acetate, Citral, Orange terpenes (natural), Cedarwood oil, alpha-Pinene, Majantol, Phenoxyethanol, Ethyl acetate, Cedrol methyl ether, 1,5,9-Trimethyl-13-oxabicyclo(10.1.0) trideca-4,8-diene, Peppermint oil, Eugenol, Ethyl maltol, Benzaldehyde, Cinnamic alcohol, 3,7-Dimethyl-1-octanol, alpha-Methyl-3,4-methylene dioxyhydrocinnamic aldehyde, beta-Pinene, d-Camphor, Methyl abietate, Cedryl acetate, Ylang ylang oil, Sandalwood oil, Mineral oil, Dimethyl benzyl carbinyl butyrate, Ethyl butyrate, Geranyl acetate, Hexylene glycol, Myrcene, alpha-Methyionantheme, beta-Ionone, 3-(4-t-Butylphenyl)propanal, 3,7-Dimethyloctan-3-yl acetate, Acetic acid, (1-oxopropoxy)-,1-(3,3-dimethylcyclohexyl), Eucalyptol, 4-Carvomenthenol, Stearic acid, Menthanyl acetate, Eucalyptus oil, Dihydroterpinyl acetate, o-t-Butylcyclohexyl acetate, Isoeugenol, alpha-Terpineol, Cyclamen aldehyde, Hydroxycitronellol, Myrcenyl acetate, Nopyl acetate, 3,7-Dimethyl-1,3,6-octatriene, Rhodinol, Dimethyl benzyl carbinyl acetate, Tricyclodecenyl propionate, 2-Methyl-5-phenylpentan-1-ol, Sclareoate, 3-Isocamphyl cyclohexanol, trans-Anethole, Hexahydro-4,7-methanoinden-5(6)-yl acetate, 4-(p-Hydroxyphenyl)-2-butanone, Nerolidol, alpha-Butylcinnamaldehyde, Bornyl acetate, Etyhl methylphenylglycidate, trans-beta-Ionone, Camphene, Juniper berry oil, Mandarin oil, Nutmeg oil, Spearmint oil, Grapefruit oil, Labdanum oil, Galbanum oil, Menthone, Trichloromethyl phenyl carbinyl acetate, alpha-Methylbenzyl acetate, Ethyl-2methyl-1,3-dioxolane-2-acetate, 2,6-Nonadienal, Abietyl acetate, A-nisic acid, Diphenyl ether, Triacetin, 2-Methyl-4-phenyl-2-butanol, Phenylethyl acetate, 1-Phenyl-3-methyl-3-pentanol, Anisyl acetate, Cinnamic aldehyde, p-Methylanisole, 5-Phenylpentanol, Diethyl malonate, Citronellal, Nerol, Undecanal,2-methyl-, Hexyl alcohol, Glyceryl caprylate, Methyl 2-nonenoate, Octyl acetate, Decanal, Lauryl alcohol, Lauric aldehyde, Ethyl vanillin, 3-Phenyl-1-propanol, Octanal, Butylated hydroxytoluene, 4-Acetyl-6-t-butyl-1,1-dimetylindane, delta-3-Caren, Benzyl laurate, Neryl acetate, Ethyl acetoacetate, Hexyl acetate, Menthol liquid, Citronellyl acetate, Tetrahydromyrcenol, Diacetin, Menthyl acetate, 3(4),8(9)-Dihydroxymethyl tricyclo(5.2.1.0(2,6)decane, 2,4-Dimethyl-3-cyclohexen-1-carboxaaldehyde, Cedrenol, Phenylacetaldehyde glyceryl acetal, Sabinene, 3,7,11-Trimethyl-1,2,10,-dodecatrien-3-ol (cis & trans), Octyldodecanol, Formaldehyde cyclododecyl ethyl acetal, Myristicin, 3,7-Dimethyl-2(3),6-nonadienenitrile, Ethyllinylyl acetate, 2-Methylbutyl acetate, cis-3-Hexenyl salicylate, 2-Methyl-4-(2,6,6-trimethyl-2(1)-cyclohexen-1-yl) butanal, Maltol isobutyrate, 2-Methyl-3(4-(2-methylpropyl)phenyl)propanal, 12-Oxahexadecanolide, 1,1-Dimethoxy-2,25-trimethyl-4-hexene, 1,6,7,8-Tetrahydro-1,4,6,6,8,8-hexamthyl-as-indacen-3<2H>-one, Bergamot oil, bergaptene free, Treemoss abs., Citrus oil distilled, Lemon terpenes, gamma-Decalactone, 2-Methyl-4-phenyl-2-pentanone, Allyl phenoxyacetate, Methyl-delta-ionone, Citronella oil, Clove bud oil, Thyme oil, Lime oil, Bois de rose oil, Cognac oil, Neroli bigarade oil, Spike lavender oil, Vetiver oil, Fir needle oil, Methylpentenolone, Lemon oil terpenes, Isobutyl salicylate, beta-Caryophyllene, Pulegone, Thymol, gamma-Terpinene, Acetyl Hexamethyl Tetralin (Moschus-Verb.), Amylacetat ,Amylsalicylat, Anethol, Anisöl, Annatto, Extrakt aus Blättern der Melisse, Öl aus Blättern der Melisse, Bayöl, Lorbeeröl, Benzaldehyd, Benzylacetat, Benzylalkohol, Benzylbenzoat, Benzyl Cinnamate (Inhaltsstoff ätherischer Öle), Benzylsalicylat, Benzylcinnamat, Calendulaöl, Kamelienöl, Kampher, Kümmelöl, Kardamomöl, Carvon, Kamillenöl, Zimtöl, Citral, Lemongrassöl, Nelkenöl, Nelkenblattöl, Cumarin, Kreuzkümmelextrakt, Dimethylbrassylat, Dipenten, Ethylvanillin, Ethylenbrassylat, Eucalyptol, Eucalyptusöl, Eugenol, Ingweröl, Gum Benzoin, Hopfenöl, Isoamylacetat, Wacholderteer, Lavendelöl, Zitronenöl, Zitronengrasöl, Liebstöckelöl, Kamillenöl, Menthol, Menthylacetat, Menthyllactat, Menthylsalicylat, Methyleugenol, Methyl Rosinate, Methyldihydrojasmonate, Muskatnussöl, Ocotea Cymbarum Öl, Weihrauchharz, Weihrauchharzextrakt, Orangenextrakt, Orangenblütenöl, Orangenblütenwasser, Orangenschalenextrakt, Petersilienöl, p-Cymen, Pentadecalacton, Pfefferminzextrakt, Pfefferminzöl, Phenethylalkohol, Kiefernöl, Kiefern(teer)öl, Rosenextrakt, Rosenöl, Rosmarinöl, Gartenrautenöl, Salbeiöl, Holunderextrakt, Holunderöl, Sandelholzöl, Sassafrasbaumöl, (süßes) Majoranöl, Teeröl, Teebaumöl, Terpineol, Thymianöl, Thymol, Vanille, Vanillin, Schafgarbenöl

Als weitere Stabilisatoren können folgende Rohstoffe eingesetzt werden:
Acetyltrifluoromethylphenylvalylglycin, Acrylamidammoniumacrylatcopolymer, Aluminummagnesiumhydroxidstearat, Ammoniumlactat, Ammoniumpolyacrylat, Ammoniumpolyacryloyldimethyltaurat, Arginin PCA, Capryloylsalicylsäureester, Zimtsäure, Cocoglucosid, Kupfergluconat, Diphenyldimethicon, Dinatriumadenosintriphosphat, Dnatriumsuccinat, Disteardimoniumhectorit, Dodecen, Eperua Falcata , hydriertes Palmenglycerid, hydriertes Palmenglyceridcitrat, Hydrierte Palmenkernglyceride, Hydrolisiertes Weizenprotein PG-Propylmethylsilandiol, Hydroxyethylacrylat / Natriumacryloyldimethyltauracopolymer, Isodeceth-6, Linseed Acid Magnesiumaspartat, Melibiose, Oxothiazolidinecarboxylsäure, Palmitoylpentapeptide 4, PEG-8 Laurat, Phenethylalkohol, Phenylpropanol, Polyacrylate-13, Polyacrylate-3, Sarcosin, Saxifraga Sarmentosa Extrakt, Scutellaria Baicalensis Extrakt, Natriummetabisulfit, Sojaisoflavon, Tocopherylglucosid, Trideceth-6, Zinkgluconat, Triacetin, 1,2 Hexandiol, Hydroxyethylpiperazine Ethane Sulfonic Acid, Nicotinamide, Penethyl Alcohol, Penthylene Glycol, Carnaubau Wax, Chlorhexidine Digluconate, Oleyl Erucate, Polycaprolactone, Sucrose Polycottonseedate, Acetyl Trifluoromethylphenyl Valylglycin, Phytol, Avena Aqua, Nylon-66, Hydroxyethylpiperazine Ethane Sulfonic Acid, Hydroxypropyl Tetrahydropyrantriol, Shorea Robusta Butter, Punica Granatum Fruit Juice, Methylserin, Ascorbyl Tocopheryl Maleate, Poly C10-30 Alkyl Acrylate, Pyracantha Fortuneana Extract, PEG-24 Cetyl Ether, Thiodipropionic Acid, Essential Oils, Tocopheryl Glucoside, PEG-24 Cholesteryl Ether, Biosaccharide Gum 3, Guanosine, Polyester-5, Dimethoxy Di-p-Cresol, Tri C14-15 Alkylcitrate, Ethyl Bisiminomethylguaiacol Manganese Chloride, Hydrated Magnesium Silicate, Lagerstroemia Indica Extract, Ethyl Glucoside, Linseed Acid, PEG / PPG-14/7 Dimethyl Ether, Sodium Dodecylbenzene Sulfonate, Isoquercitrin, Thiotaurine, Andrographolide, Erythritol, Xymenynic Acid, Coffea Robusta, Disodium NADH, Lauryl Dimonium Hydroxypropyl Hydrolyzed Soy Protein, Methylsilanol / Silicate Crosspolymer, Amaranthus Caudatus Extract, BGT, Chrysanthenum Parthenium, Lauryl Polyglucoside, Sodium Hydroxypropyl Starch Phosphate, PEG-60 Glyceryl Isostearate, Glucosylrutin, Mineral Water, Pisces Collagen, Sodium Hydroxyethyl Acrylate / Acryloyldimethyl Taurate Copolymer, Sodium Mannose Phosphate, Tetrahydrobisdemethoxycurcumin, Tetrahydrodemethoxycurcumin, Tetrahydrodemethoxydiferuloylmethan, Butylhydrochinone, Citrus Aurantium Dulcis Blossoms, Hierochloe Odorata Extract, Kaempferia Galanga Root Extract, PEG-2 Stearyl Ether, Succinoglycan, Trioctyldodecyl Citrate, Coleus Barbatus Extract, Eclipta Prostrata Extract, PEG-10 Dimethicone / Vinyl Dimethicone Crosspolymer, Polyglyceryl-2 Caprate, Pyrus Malus Water, Trioxaundecanedioic Acid, Amaranthus Extract, Dodecene, Hydrolyzed Cera Alba, Oxothiazolidinecarboxylic Acid, PEG-20 Stearyl Ether, Platanus Occidentalis, Selaginella Tamariscina Extract, Tetrahydrobisdemethoxydiferuloylmethane, Anthemis Nobilis Flower, Cassia Alata, Echium Lycopsis Oil, Eucalyptol, Heptapeptide-6, Humulus Japonicus Flower / Leaf / Stem Extract, Hydrogenated Myristyl Olive Esters, Hydrolyzed Wheat Protein PG-Propyl Methylsilanediol, Lupinus Luteus Extract, Palmitoyl Lysyl Aminovaleroyl Lysine, Palmitoyl Tetrapeptide-10, Polyglyceryl-6 Polyricinoleate

Bevorzugt im Sinne der vorliegenden Erfindung sind Pigmentmischungen aus Weiß-Pigmenten (z. B. Kaolin, Titandioxid oder Zinkoxid) und anorganischen Farbpigmenten (z. B. Eisenoxid-Pigmente, Chromoxide), wobei die Pigmente beschichtet ("gecoatet") oder unbeschichtet vorliegen können. Unter den Farbpigmenten sind Eisenoxide besonders bevorzugt. Die erfindungsgemäßen Pigmente können anorganisch oder organisch sein.

Weißpigmente sind Pigmente, deren optische Wirkung vorwiegend auf nicht-selektiver Lichtstreuung beruht (siehe auch DIN 55944: 2003-11). Von den chemisch oft sehr ähnlichen Füllstoffen unterscheiden sich anorganische Weißpigmente vor allem durch ihre im Allgemeinen höhere Brechzahl und - damit verbunden - ihr höheres Streuvermögen sowie nach DIN 55943: 2001-10 durch ihre Anwendung.

Erfindungsgemäß bevorzugte Weißpigmente zeigen keine Absorption im Bereich des sichtbaren Lichts, dafür aber ein hohes Streuvermögen, welches ein hohes Deckvermögen zur Folge hat. Das Streuvermögen ist umso größer, je größer die Differenz zwischen der Brechzahl des Weißpigmentes und der des umgebenden Mediums ist.

Erfindungsgemäß vorteilhafte Weißpigmente sind Titandioxide (Brechzahlen: 2,55 für Anatas und 2,75 für Rutil) und Zinkoxide (Brechzahl zwischen 1,95 und 2,1). Besonders bevorzugt ist Titandioxid.

Vorteilhaft im Sinne der vorliegenden Erfindung können das oder die Pigmente auch aus der Gruppe der Effektpigmente gewählt werden, welche der kosmetischen Zubereitung neben der reinen Farbe eine zusätzliche Eigenschaft - wie z. B. eine Winkelabhängigkeit der Farbe (changieren, Flop), Glanz (nicht Oberflächenglanz) oder Textur - verleihen. Solche Effektpigmente werden erfindungsgemäß vorteilhaft zusätzlich zu einem oder mehreren Weiß- und/oder Farbpigmenten eingesetzt.

Die bedeutendste Gruppe der Effektpigmente stellen die Glanzpigmente dar, zu denen nach DIN 55944: 2003-11 die Metalleffektpigmente und die Perlglanzpigmente gehören. Einige spezielle Effektpigmente lassen sich diesen beiden Gruppen nicht zuordnen, z. B. plättchenförmiges Graphit, plättchenförmiges Eisenoxid und mikronisiertes Titandioxid, wobei mikronisiertes Titandioxid keinen Glanzeffekt, sondern einen winkelabhängigen Lichtstreueffekt erzeugt. Bei den Glanzpigmenten nach DIN 55943: 2001-10 handelt es sich vorwiegend um plättchenförmige Effektpigmente. Parallel orientiert zeigen Glanzpigmente einen charakteristischen Glanz. Die optische Wirkung von Glanzpigmenten beruht auf der gerichteten Reflexion an metallischen Teilchen (Metalleffektpigmente), an transparenten Teilchen mit hoher Brechzahl (Perlglanzpigmente) oder auf dem Phänomen der Interferenz (Interferenzpigmente) (DIN 55944: 2003-11).

Beispiele für erfindungsgemäß bevorzugte handelsübliche Effektpigmente sind: Timiron® von Merck, Iriodin® von Merck (Perl- und Farbglanzpigmente für dekorative technische Anwendungen), Xirallic® von Merck (farbintensive Kristalleffektpigmente).

Ferner können die erfindungsgemäßen Zubereitungen vorteilhaft auch organische Farbpigmente enthalten, d. h. organische Farbstoffe, welche in der Zubereitung praktisch unlöslich sind. Nach DIN 55944: 1990-04 können organische Pigmente nach chemischen Gesichtspunkten in Azopigmente und polycyclische Pigmente sowie nach farblichen Gesichtspunkten in Bunt- oder Schwarzpigmente eingeteilt werden. Organische Weißpigmente sind ohne praktische Bedeutung.

Die Pigmente können vorteilhaft im Sinne der vorliegenden Erfindung auch in Form kommerziell erhältlicher öliger oder wässriger Vordispersionen zur Anwendung kommen.

Es ist ferner vorteilhaft im Sinne der vorliegenden Erfindung, wenn die erfindungsgemäße Zubereitung eine oder mehrere Farbstoffe enthält.

Die Farbstoffe können sowohl synthetischen als auch natürlichen Ursprungs sein.

Als Farbstoffe können die für kosmetische Zwecke geeigneten und zugelassenen Substanzen verwendet werden. Beispiele sind Kochenillerot A (C.I. 16255), Patentblau V (C.I.42051), Indigotin (C.I.73015), Chlorophyllin (C.I.75810), Chinolingelb (C.I.47005). Titandioxid (C.I.77891), Indanthrenblau RS (C.I. 69800) und Krapplack (C.I.58000).

Diese Farbstoffe werden üblicherweise in Konzentrationen von 0,001 bis 1 Gew.-%, bezogen auf die gesamte Mischung, formuliert.

Die erfinderischen Zubereitungen können auch Repellentien enthalten.

Die meisten Repellent-Wirkstoffe gehören den Stoffklassen der Amide, Alkohole, Ester und Ether an.

Ein moderner Repellent-Wirkstoff ist beispielsweise der 1-Piperidincarboxylsäure 2-(2-hydroxyethyl)-1-methylpropylester (INN: Icaridin, CAS-Nummer: 119515-38-7, Elincs-Nummer: 423-210-8), der die folgende Struktur aufweist:

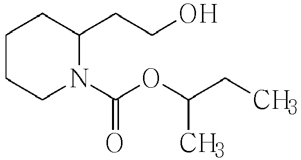

Ein weiterer häufig eingesetzter Repellent-Wirkstoff ist der 3-(N-n-Butyl-N-acetyl-amino)-propionsäureethylester (auch als Ethylbutylacetylaminopropionat oder Repellent 3535 bezeichnet), welcher sich durch die folgende Strukturformel auszeichnet:

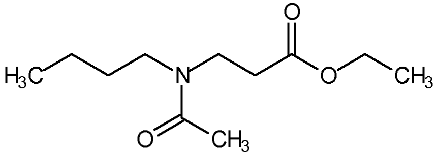

Nicht zuletzt kennt der Fachmann den Repellent-Wirkstoff N,N-Diethyl-3-methylbenzamid (Handelsbezeichnung: Meta-delphene, DEET), der di folgende Struktur aufweist:

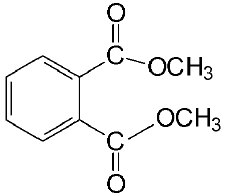

Zu den erfindungsgemäßen Formulierungen können auch Wirkstoffe hinzugefügt werden.

Unter den Wirkstoffen sind beispielsweise Tocopherol, Tocopherolacetat, Tocopherolpalmitat, Ascorbinsäure, (Desoxy)Ribonucleinsäure und deren Fragmentierungsprodukte, β-Glucane, Retinol, Bisabolol, Allantoin, Phytantriol, Panthenol, AHA-Säuren, Aminosäuren, Ceramide, Pseudoceramide, essentielle Öle, Pflanzenextrakte, wie z. B. Prunusextrakt, Bambusextrakt, Lindenblütenextrakt und Vitaminkomplexe zu verstehen.

In einer bevorzugten Ausführungsform der Erfindung enthaltend die erfindungsgemäßen Zubereitungen mindestens eine Verbindung ausgewählt aus Vitaminen, Allantoin, Bisabolol, Glycerylglucose und Pflanzenextrakten.

In einer bevorzugten Ausführungsform der Erfindung enthaltend die erfindungsgemäßen Zubereitungen mindestens eine Verbindung ausgewählt aus Tocopherol, Tocopherolacetat, Tocopherolpalmitat, Ascorbinsäure, Harnstoff, β-Glucane, Retinol, Bisabolol, Allantoin, Phytantriol, Panthenol, AHA-Säuren, Pflanzenextrakten und Mischungen daraus.

Die folgenden Beispiele sollen die Erfindung erläutern, aber nicht einschränken.

| **Beispielrezeptur 1** | **Gew.-%** |
|---|---|
| Stearinsäure | 2 |
| Glycerylstearat | 2 |
| Cetylalkohol | 2 |
| Isohexadecan | 2 |
| Dicaprylylether | 4 |
| C12-15 Alkylbenzoat | 4 |
| Myristylmyristat | 1 |
| Sonnenblumenöl | 1 |
| Süßholzextrakt | 0,1 |
| Carrageenan | 0,2 |
| Carbomer | 0,3 |
| Methylparaben | 0,4 |
| 4-Hydroxyacetophenon | 0,7 |
| Glycerin | 5 |
| EDTA | 0,2 |
| Parfüm | 0,2 |
| Natronlauge (pH 7,5 eingestellt) | q.s. |
| Wasser | ad 100 |

| **Beispielrezeptur 2** | **Gew.-%** |
|---|---|
| Cetylstearylalkohol | 3 |
| Caprylsäure/Caprinsäuretriglyceride | 3 |
| Dicaprylylcarbonat | 3 |
| Dicaprylylether | 2 |
| Octocrylen | 5 |
| Butylmethoxydibenzoylmethan | 1 |
| Sodium Stearoylglutamat | 0,3 |
| 4-Hydroxyacetophenon | 0,4 |
| Acrylat/C10-30 Alkyl Acrylat Crosspolymer | 0,4 |
| Xanthan Gum | 0,2 |
| Nachtkerzensamenöl | 1 |
| Vitamin A palmitat | 0,1 |
| Glycerin | 6 |
| Phenoxyethanol | 0,5 |
| Natronlauge (pH 6 eingestellt) | q.s. |
| Wasser 100% | ad 100 |

| **Beispielrezeptur 3** | **Gew.-%** |
|---|---|
| Cetylstearylalkohol | 3 |
| Caprylsäure/Caprinsäuretriglyceride | 3 |
| Dicaprylylcarbonat | 3 |
| Dicaprylylether | 2 |
| Ethlyhexylmethoxycinnamat | 4 |
| Butylmethoxydibenzoylmethan | 1 |
| Sodium Stearoylglutamat | 0,3 |
| 4-Hydroxyacetophenon | 0,5 |
| Acrylat/C10-30 Alkyl Acrylat Crosspolymer | 0,4 |
| Xanthan Gum | 0,2 |
| Organischer Farbstoff, wasserlöslich | 0,1 |
| Niacinamid | 0,2 |
| Glycerin | 5 |
| Ethylparaben | 0,1 |
| Phenoxyethanol | 0,5 |
| Parfum | 0,3 |
| Natronlauge (pH 6 eingestellt) | q.s. |
| Wasser 100% | ad 100 |

### Gesichtscreme

| **Beispielrezeptur 4** | **Gew.-%** |
|---|---|
| Glycerylstearat | 2 |
| Stearylalkohol | 2 |
| Caprylsäure/Caprinsäuretriglyceride | 3 |
| Dicaprylylether | 3 |
| C12-15 Alkylbenzoat | 3 |
| Kalium Cetylphosphat | 0,3 |
| Camellia sinensis Extrakt (Grüner Tee) | 0,1 |
| Tris-Biphenyl Triazin | 1 |
| Diethylhexylbutamidotriazon | 1 |
| Ethylhexyltriazon | 1 |
| Terephthaliden Dicampher Sulfonsäure (Natriumsalz) | 0,5 |
| Ammonium Acryloyldimethyltaurat/VP Copolymer (Aristoflex AVC®) | 0,4 |
| Xanthan Gum | 0,1 |
| 4-Hydroxyacetophenon | 0,3 |
| Traubenkernöl | 0,5 |
| Rucinol | 0,1 |
| Glycerin | 7 |
| EDTA | 0,2 |
| Konservierung | q.s. |
| Natronlauge (pH 7,0 eingestellt) | q.s. |
| Wasser 100% | ad 100 |

| **Beispielrezeptur 5** | **Gew.-%** |
|---|---|
| Glycerylstearat | 2 |
| Stearylalkohol | 1 |
| Caprylsäure/Caprinsäuretriglyceride | 2 |
| Dicaprylylcarbonat | 3 |
| Dicaprylylether | 4 |
| Sodium Stearoylglutamat | 0,3 |
| Organischer Farbstoff, öllöslich, blau | 0,05 |
| 4-Hydroxyacetophenon | 0,3 |
| Acrylat/C10-30 Alkyl Acrylat Crosspolymer | 0,3 |
| Xanthan Gum | 0,2 |
| Ascorbinsäure | 0,1 |
| Glycerin | 6 |
| Methylparaben | 0,2 |
| Phenoxyethanol | 0,5 |
| Parfum | 0,4 |
| Natronlauge (pH 6 eingestellt) | q.s. |
| Wasser 100% | ad 100 |

## Patentansprüche

1. O/W-Emulsionen enthaltend
(a) 4-Hydroxyacetophenon und
(b) anionische Emulgatoren ausgewählt aus der Gruppe, die gebildet wird von Acylglutamaten, Acylpeptiden, Sarcosinaten, Tauraten, Acyllactylaten, Alaninaten, Carbonsäuren, Ester-Carbonsäuren, Ether-Carbonsäuren, Acylisethionaten, Alkylarylsulfonaten, Alkylsulfonaten, Sulfosuccinaten, Alkylethersulfaten, Alkylsulfaten, Alkyletherphosphaten und deren Mischungen.

2. O/W-Emulsionen nach Anspruch 1, **dadurch gekennzeichnet, dass** die anionischen Emulgatoren ausgewählt sind aus der Gruppe, die gebildet wird von Stearoylglutamat-Alkalisalzen, Natrium Cetylsulfat und Stearinsäure sowie ihren Alkalisalzen.

3. O/W-Emulsionen nach den Ansprüchen 1 und/oder 2, **dadurch gekennzeichnet, dass** sie die Komponente (a) in Mengen von 0,01 bis 10 Gew.-% - bezogen auf das Gesamtgewicht - enthalten.

4. O/W-Emulsionen nach mindestens einem der Ansprüche 1 bis 3, **dadurch gekennzeichnet, dass** sie weiterhin Farbstoffe, Parfümbestandteile, kosmetische Duftkomponenten, und/oder kosmetische oder dermatologische Wirkstoffe enthalten.

5. Verwendung von 4-Hydroxyacetophenon zur Stabilisierung von
(i) Farbstoffen,
(ii) Parfümbestandteilen oder kosmetischen Duftkomponenten, und/oder
(iii) kosmetischen oder dermatologischen Wirkstoffen
in O/W-Emulsionen gegen chemische Zersetzung durch Temperatur- oder Lichteinwirkung.

## Claims

1. O/W emulsions comprising
(a) 4-hydroxyacetophenone and
(b) anionic emulsifiers selected from the group consisting of acyl glutamates, acyl peptides, sarcosinates, taurates, acyl lactates, alanates, carboxylic acids, ester carboxylic acids, ether carboxylic acids, acyl isethionates, alkyl aryl sulfonates, alkyl sulfonates, sulfosuccinates, alkyl ether sulfates, alkyl sulfates, alkyl ether phosphates and their mixtures.

2. O/W emulsions according Claim 1, **characterized in that** anionic emulsifiers are selected from the group consisting of stearoyl glutamate alkali salts, sodium cetyl sulfate and stearic acid and their alkali salts.

3. O/W emulsions according to Claims 1 and/or 2, **characterized in that** component (a) is present in amounts of from 0.01 to 10 wt.-%.

4. O/W emulsions according to any of Claims 1 to 3, **characterized in that** they further comprise dyes, perfume compounds, cosmetic fragrance compounds and/or cosmetic or dermatologic agents.

5. Use of 4-hydroxyacetophenone for stabilization of
(i) dyes,
(ii) perfume components or cosmetic fragrance components and/or
(iii) cosmetic and/or dermatologic agents
in o/w emulsions against chemical degradation effected by temperature or light.

## Revendications

1. Émulsions H/E, contenant :
(a) de la 4-hydroxyacétophénone et
(b) des émulsifiants anioniques choisis dans le groupe formé par les acylglutamates, les acylpeptides, les sarcosinates, les taurates, les acyllactylates, les alaninates, les acides carboxyliques, les esters-acides carboxyliques, les éthers-acides carboxyliques, les acyliséthionates, les alkylarylsulfonates, les alkylsulfonates, les sulfosuccinates, les alkyléthersulfates, les alkylsulfates, les alkylétherphosphates et leurs mélanges.

2. Émulsions H/E selon la revendication 1, **caractérisées en ce que** les émulsifiants anioniques sont choisis dans le groupe formé par les sels alcalins de glutamate de stéaroyle, le cétylsulfate de sodium et l'acide stéarique, ainsi que ses sels alcalins.

3. Émulsions H/E selon les revendications 1 et/ou 2, **caractérisées en ce qu'**elles contiennent le composant (a) en quantités de 0,01 à 10 % en poids, par rapport au poids total.

4. Émulsions H/E selon au moins l'une quelconque des revendications 1 à 3, **caractérisées en ce qu'**elles contiennent en outre des colorants, des constituants parfums, des composants odorants cosmétiques et/ou des agents actifs cosmétiques ou dermatologiques.

5. Utilisation de 4-hydroxyacétophénone pour la stabilisation
(i) de colorants,
(ii) de constituants parfums ou de composants odorants cosmétiques et/ou
(iii) d'agents actifs cosmétiques ou dermatologiques
dans des émulsions H/E contre la décomposition chimique sous l'effet de la température ou de la lumière.
